# EUROPEAN PATENT SPECIFICATION

(11) **EP 2 495 241 B1**
(45) Date of publication and mention of the grant of the patent: **04.06.2014**
(21) Application number: 10825966.4
(22) Date of filing: 10.04.2010
(51) Int. Cl.: C07D 307/93, B01J 23/80

(54) **ENDO-METHYLENE HEXAHYDROPHTHALIC ANHYDRIDE AND PRODUCTION METHOD THEREOF**
ENDOMETHYLEN-HEXAHYDROPHTHAL-ANHYDRID UND HERSTELLUNGSVERFAHREN DAFÜR
ANHYDRIDE ENDO-MÉTHYLÈNEHEXAHYDROPHTALIQUE ET SON PROCÉDÉ DE PRODUCTION

(30) Priority: 30.10.2009 CN 200910066315
(43) Date of publication of application: 05.09.2012
(73) Proprietor: Puyang Huicheng Chemicals Co., Ltd, Puyang City, Henan 457001 (CN)
(72) Inventor: YANG, Zhenqiang, Zhengzhou Henan 450002 (CN); ZHANG, Haiyang, Zhengzhou Henan 450002 (CN); LV, Haikuan, Zhengzhou Henan 450002 (CN); CHEN, Shumin, Zhengzhou Henan 450002 (CN); HAN, Zhaohai, Zhengzhou Henan 450002 (CN); ZHANG, Pengke, Puyang City Henan 457001 (CN); CUI, Fumin, Puyang City Henan 457001 (CN); WANG, Zhongfeng, Puyang Henan 457001 (CN); YANG, Ruina, Zhengzhou Henan 450002 (CN); WANG, Guoqing, Puyang City Henan 457001 (CN); WANG, Tianze, Puyang City Henan 457001 (CN); LIU, Fagui, Puyang City Henan 457001 (CN)
(74) Representative: Stolmár & Partner
(86) International application number: PCT/CN2010/071679
(87) International publication number: WO 2011/050602

(56) References cited:
- WO-A1-02/066412
- CN-A- 1 501 905
- CN-A- 1 845 892
- CN-A- 101 481 368
- PERSEPHONE CANONNE ET AL: "Novel synthesis of five- and six-membered spiro .gamma.-lactones in rigid bicyclic systems", THE JOURNAL OF ORGANIC CHEMISTRY, vol. 47, no. 20, 1 September 1982 (1982-09-01), pages 3953-3959, XP55058258, ISSN: 0022-3263, DOI: 10.1021/jo00141a028
- SHI, QIUJIE ET AL.: 'Effect of Co and Mo Dopant on the Properties of Ni/ZnO-ZrO2 for Hydrodesulfurization of Thiophene' JOUMAL OF MOLECULAR CATALYSIS vol. 23, no. 2, April 2009, CHINA, pages 130 - 134, XP008156457

## Description

### Field of the Invention

The present invention relates to a chemical product and a method producing thereof, in particularly hexahydro-3,6-methanophthalic anhydride and a method producing thereof, and belongs to the field of organic chemical synthesis.

### Background of the Invention

5-norbomene-2,3-dicarboxylic anhydride (Nadic anhydride, Himic anhydride) is an important chemical raw material widely used for materials of electronic information, synthetic resins and plastics, pesticide and pharmacy, and so on; and can be prepared with simple processes and low material costs. In addition, resins synthesized by using thereof as a raw material have better air-drying property, higher thermal resistance, better surface finish, and improved electricity property, erosion resistance and mechanical intensity than resins synthesized by hexahydrophthalic anhydride and tetrahydrophthalic anhydride. The hexahydro-3,6-methanophthalic anhydride is a product after hydrogenation of a Nadic anhydride. Comparing with Nadic anhydride, hexahydro-3,6-methanophthalic anhydride has a more stabilized chemical structure and physical/chemical property and lower viscosity, and the product thereof has a lighter solid color and is more weather resistance. Therefore, it can be used to replace Nadic anhydride. The development of petroleum industry in China also increases the quantity of cyclopentadiene, a byproduct of ethylene, but most of which is not fully developed and used. To solve the problem of comprehensive use of cyclopentadiene as well as to provide hexahydro-3,6-methanophthalic anhydride as the raw material having widely potential applications to chemical industry, it is important to carry out studies on its synthesizing method.

WO 02/066412 A1 discloses a method for producing cyclohexane dicarboxylic acids or derivatives thereof, e.g esters and anhydrides, wherein diene maleic acid anhydride mixtures, especially mixtures of butadiene maleic acid anhydrides or mixtures of maleic acid anhydride and C5 dienes, are reacted to form alkyl substituted or unsubstituted cyclohexene dicarboxylic acid anhydrides in a condensed phase; esters are formed and hydrogenation is carried out to obtain a corresponding cyclohexane dicarboxylic acid derivative. WO 02/066412 A1 also discloses the use of cyclohexane dicarboxylic acids or derivatives produced according to the described method as softeners for plastics.

### Contents of the Invention

The object of the present invention is to provide a method for the production of hexahydro-3,6-methanophthalic anhydride through two steps of reaction using mixed C₅s as a raw material, resulting in low production costs and less pollutions; another object of the invention is to provide a new chemical raw material, i.e., hexahydro-3,6-methanophthalic anhydride.

For achieving the purpose of the present invention, the technical solution of the present invention is as below:

Maleic anhydride and mixed C₅s (major components thereof are cyclopentadiene, isoprene and 1,3-pentadiene) are used as raw materials to produce Nadic anhydride by absorbing cyclopentadiene with maleic anhydride directly at 0-5°C, and obtains Nadic anhydride pure product through crystallization. Then hexahydro-3,6-methanophthalic anhydride is prepared through catalytic hydrogenation.

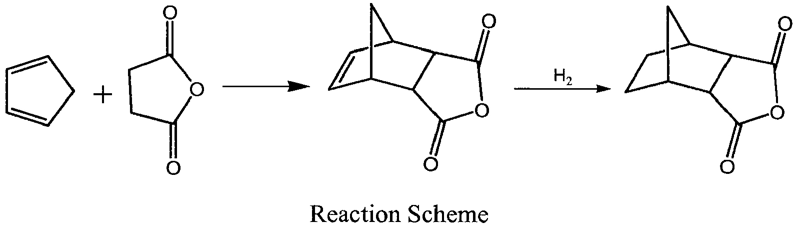

The specific reaction steps are as below:

Adding maleic anhydride to mixed C₅s gradually with stirring, controlling a reaction temperature at 0-5°C, terminating the reaction when no cyclopentadiene is detected by gas chromatography, separating via centrifuge, vaporizing, compressing, and condensing the liquid to obtain de-cyclized C₅s having isoprene and 1,3-pentadiene as major components, crystallizing the solid to obtain Nadic anhydride. Then adding a catalyst Cu-Ni/ZnO-ZrO₂ or Co-Ni/ZnO-ZrO₂ to the obtained Nadic anhydride, wherein a content ofNi is 10-30% by mass (not including the doping amounts of Cu, Co and ZnO), a molar ratio of ZnO to ZrO₂ is preferably 1:20-50, a molar ratio of Cu to Ni is preferably 1:100-200, and a molar ratio of Co to Ni is preferably 1:50-200. Carrying out a catalytic hydrogenation reaction for 4-6 hours in a high-pressure autoclave at a temperature of 100-140°C and under a hydrogen pressure of 1-4 MPa, filtering the catalyst after cooling, and reduced pressure distilling to obtain the hexahydro-3,6-methanophthalic anhydride with a yield of 90-92%.

Preparation of the Cu-Ni/ZnO-ZrO₂ catalyst: drop-adding ammonia water slowly to a mixed solution of ZrOCl₂·8H₂O, Zn(NO₃)₂·6H₂O, Ni(NO₃)₂·6H₂O and Cu(NO₃)₂·3H₂O in a water bath at a constant temperature of 25°C with stirring, aging overnight after the solution is deposited completely, then filtrating, and washing with deionized water to obtain a neutralized pH. After drying, a precursor of Cu-Ni/ZnO-ZrO₂ catalyst is obtained, then baked at 400-500°C, and subjected to a reduction to obtain a Cu-Ni/ZnO-ZrO₂ catalyst. During synthesis, a preferable molar ratio of Cu(NO₃)₂·3H₂O to Ni(NO₃)₂·6H₂O is 1:100-200; a preferable molar ratio of Zn(NO₃)₂·6H₂O to ZrOCl₂·8H₂O is 1:20-50; and a preferable molar ratio of Ni(NO₃)₂·6H₂O to ZrOCl₂·8H₂O is 1:1.23-4.75.

Preparation of the Co-Ni/ZnO-ZrO₂ catalyst: drop-adding ammonia water slowly to a mixed solution of ZrOCl₂·8H₂O, Zn(NO₃)₂·6H₂O, Ni(NO₃)₂·6H₂O and Co(NO₃)₂·6H₂O in water bath at a constant temperature of 25°C with stirring, aging overnight after the solution is deposited completely, then filtrating, and washing with deionized water to obtain a neutralized pH. After drying, a precursor of Co-Ni/ZnO-ZrO₂ catalyst is obtained, then baked at 450-550°C, and subjected to a reduction to obtain a Co-Ni/ZnO-ZrO₂ catalyst. During synthesis, a preferable molar ratio of Co(NO₃)₂·6H₂O to Ni(NO₃)₂·6H₂O is 1:50-200; a preferable molar ratio of Zn(NO₃)₂·6H₂O to ZrOCl₂·8H₂O is 1:20-50; and a preferable molar ratio of Ni(NO₂)₂·6H₂O to ZrOCl₂·8H₂O is 1:1.23-4.75.

The beneficial effects of the present application include: (1) mixed C₅s can be used directly as the raw material in the method and there is no need to perform an operation of refinement and separation to the raw material, which reduces production costs dramatically, meanwhile, cyclopentadiene in the mixed C₅s is removed effectively after reaction, and the de-cyclized C₅s can be sold as a new chemical material; (2) the method uses a composite catalyst Cu-Ni/ZnO-ZrO₂ or Co-Ni/ZnO-ZrO₂, which reduces polymerization of Nadic anhydride inside the system and avoids reduction of a carbonyl group in the anhydride, and the catalyst is a heterogeneous catalyst which may be separated easily, which reduces the separation cost of the catalyst; (3) the raw material can be fully used, while using mixed C₅s, an inexpensive byproduct of ethylene, as a raw material to prepare hexahydro-3,6-methanophthalic anhydride, another new chemical material, de-cyclized C₅s, is obtained at the same time, which enhances the in-depth processing of the mixed C₅s, reduces environmental pollution, and facilitates industrial production, and is extremely practical.

### Description of Drawings

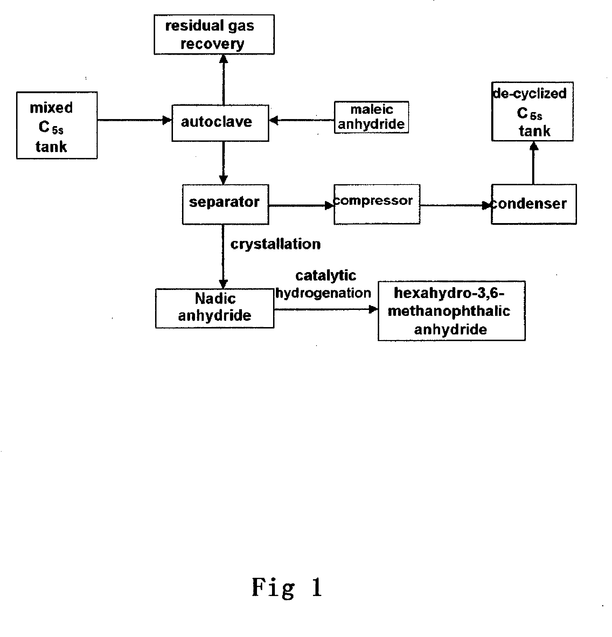

Figure 1 is a processing flow chart of the method.

### Specific Mode for Carrying Out the Invention

The following examples are for illustrating the present invention in more details:

### Example 1

Adding maleic anhydride to 600 g of mixed C₅s gradually at 0°C with stirring, terminating the reaction when no cyclopentadiene is detected by gas chromatography, adding totally 209 g of maleic anhydride, filtrating, crystallizing the solid to obtain 290 g of Nadic anhydride, with a yield of 83%.

Then adding 8.7g of the Cu-Ni/ZnO-ZrO₂ catalyst to the generated Nadic anhydride (having a Ni content of 10% and a molar ratio of Cu to Ni of 1:100), carrying out a catalytic hydrogenation reaction for 4 hours in a high-pressure autoclave at a temperature of 140°C and under a hydrogen pressure of 3 MPa, filtering the catalyst after cooling, reduced pressure distilling to obtain 261 g of hexahydro-3,6-methanophthalic anhydride, with a yield of 90%.
¹H NMR (300 MHz, CDCl₃, exo-), δ/ppm: 2.92-2.93(m, 2H, H-2, H-3), 2.83(m, 2H, H-1, H-4), 1.28-1.70(m, 6H);
¹H NMR (300 MHz, CDCl₃, endo-), δ/ppm: 3.41-3.43(m, 2H, H-2, H-3), 2.84(m, 2H, H-1, H-4), 1.32-1.73(m, 6H);
¹³C NMR (300 MHz, CDCl₃, exo-), δ/ppm: 173.1, 49.0, 42.3, 40.9, 27.3;
¹³C NMR (300 MHz, CDCl₃, endo-), δ/ppm: 172.3, 49.8, 40.1, 34.3, 24.9;
IR (KBr), v/cm⁻¹: 2948, 1894, 1867, 1787, 1775, 1267, 1221, 1030, 972, 907;
Anal. Calcd for C₉H₁₀O₃: C, 65.05 ; H, 6.07. Found: C, 65.25 ; H, 6.15;
Ms(m/z): 166.2(M⁺).

### Example 2

Adding maleic anhydride to 6 Kg of mixed C₅s gradually at a temperature of 2°C with stirring, terminating the reaction when no cyclopentadiene is detected by gas chromatography, adding totally 2.1 Kg of maleic anhydride, separating a reacting mixture via centrifuge, vaporizing, compressing, and condensing the liquid to obtain 4.8 Kg of de-cyclized C₅s, crystallizing the solid to obtain 2.6 Kg ofNadic anhydride, having a yield of 73%.

Then adding 78 g of the Co-Ni/ZnO-ZrO₂ catalyst (having a Ni content of 20% and a molar ratio of Co to Ni of 1:50) to the generated Nadic anhydride, carrying out a catalytic hydrogenation reaction in a high-pressure autoclave for 5 hours at a temperature of 110°C and under a hydrogen pressure of 4 MPa, filtering the catalyst after cooling, reduced pressure distilling to obtain 2.4 Kg of hexahydro-3,6-methanophthalic anhydride, with a yield of 92%.

### Example 3

Adding maleic anhydride to 18 Kg of mixed C₅s gradually at a temperature of 5°C with stirring, terminating the reaction when no cyclopentadiene is detected by gas chromatography, adding totally 6.3 Kg of maleic anhydride, separating a reaction mixture via centrifuge, vaporizing, compressing, and condensing the liquid to obtain 13.8 Kg of de-cyclized C₅s, crystallizing the solid to obtain 8.4 Kg of Nadic anhydride, with a yield of 79%.

Then adding 252g of the Cu-Ni/ZnO-ZrO₂ catalyst to the generated Nadic anhydride (having a Ni content of 30% and a molar ratio of Cu to Ni of 1:200), carrying out a catalytic hydrogenation reaction in a high-pressure autoclave for 6 hours at a temperature of 130°C and under a hydrogen pressure of 1 MPa, filtering the catalyst after cooling, reduced pressure distilling to obtain 7.5 Kg of hexahydro-3,6-methanophthalic anhydride, with a yield of 90%.

### Example 4

Adding maleic anhydride to 12 Kg of mixed C₅s gradually at a temperature of 5°C with stirring, terminating the reaction when no cyclopentadiene is detected by gas chromatography, adding totally 4.2 Kg of maleic anhydride, separating a reaction mixture via centrifuge, vaporizing, compressing, and condensing the liquid to obtain 9.2 Kg of de-cyclized C₅s, crystallizing the solid to obtain 5.6 Kg of Nadic anhydride, with a yield of 79%.

Then adding 168 g of the Co-Ni/ZnO-ZrO₂ catalyst to the generated Nadic anhydride (having a Ni content of 30% and a molar ratio of Co to Ni of 1:200); carrying out a catalytic hydrogenation reaction in a high-pressure autoclave for 6 hours at a temperature of 120°C and under a hydrogen pressure of 1 MPa, filtering the catalyst after cooling, reduced pressure distilling to obtain 5.1 Kg of hexahydro-3,6-methanophthalic anhydride, with a yield of 91 %.

## Claims

1. A method for production of hexahydro-3,6-methanophthalic anhydride having the following structural formula: 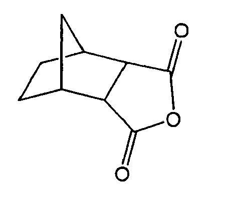 **characterized in that** the method comprises the following steps: adding maleic anhydride to mixed C₅s gradually with stirring, controlling a reaction temperature at 0-5°C, terminating the reaction when no cyclopentadiene is detected through gas detection, separating via centrifuge, vaporizing, compressing, and condensing the liquid to obtain de-cyclized C₅s having isoprene and 1,3-pentadiene as major components, crystallizing the solid to obtain Nadic anhydride; then adding a catalyst Cu-Ni/ZnO-ZrO₂ or Co-Ni/ZnO-ZrO₂ to the obtained Nadic anhydride, carrying out a catalytic hydrogenation reaction for 4-6 hours in a high-pressure autoclave at a temperature of 100-140°C and under a hydrogen pressure of 1-4 MPa, filtering the catalyst after cooling, and reduced pressure distilling to obtain the hexahydro-3,6-methanophthalic anhydride.

2. The method for production of hexahydro-3,6-methanophthalic anhydride according to claim 1, **characterized in that** cyclopentadiene, 1,3-pentadiene and isoprene are major components of mixed C₅s.

3. The method for production of hexahydro-3,6-methanophthalic anhydride according to claim 1, **characterized in that** in the catalyst Cu-Ni/ZnO-ZrO₂ or Co-Ni/ZnO-ZrO₂, a content of Ni is 10-30 % by mass excluding doping amounts of Cu, Co and ZnO, a molar ratio of ZnO to ZrO₂ is preferably 1:20-50, a molar ratio of Cu to Ni is 1:100-200, and a molar ratio of Co to Ni is 1:50-200.

## Patentansprüche

1. Verfahren zur Herstellung von Hexahydro-3,6-methanophthalsäureanhydrid der Strukturformel 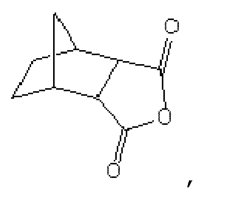 **dadurch gekennzeichnet, dass** das Verfahren folgende Schritte umfasst: Zugeben von Maleinsäureanhydrid zu einem C₅-Gemisch schrittweise unter Rühren, Einstellen der Reaktionstemperatur bei 0-5 °C, Beenden der Reaktion, wenn kein Cyclopentadien mehr mittels Gasdetektion nachgewiesen wird, Abtrennen über eine Zentrifuge, Vaporisieren, Einengen und Kondensieren der Flüssigkeit, um ein C₅-Gemisch zu erhalten, das frei von cyclischen Komponenten ist und das Isopren und 1,3-Pentadien als Hauptkomponenten aufweist, Auskristallisieren des Feststoffs, um 5-Norbornen-2,3-dicarbonsäureanhydrid (Nadic anhydrid) zu erhalten; sodann Zugeben eines Cu-Ni/ZnO-ZrO₂- oder Co-Ni/ZnO-ZrO₂-Katalysators zu dem erhaltenen 5-Norbornen-2,3-dicarbonsäureanhydrid, Durchführen einer katalytischen Hydrierungsreaktion für 4-6 Stunden in einem Hochdruckautoklaven bei einer Temperatur von 100-140 °C und unter einem Wasserstoffdruck von 1-4 MPa, Abfiltrieren des Katalysators nach dem Abkühlen, und Destillieren unter vermindertem Druck, um das Hexahydro-3,6-methanophthalsäureanhydrid zu erhalten.

2. Verfahren zur Herstellung von Hexahydro-3,6-methanophthalsäureanhydrid gemäß Anspruch 1, **dadurch gekennzeichnet, dass** Cyclopentadien, 1,3-Pentadien und Isopren die Hauptkomponenten des C₅-Gemischs sind.

3. Verfahren zur Herstellung von Hexahydro-3,6-methanophthalsäureanhydrid gemäß Anspruch 1, **dadurch gekennzeichnet, dass** der Cu-Ni/ZnO-ZrO₂- oder Co-Ni/ZnO-ZrO₂-Katalysator einen Ni-Anteil von 10-30 m.-% aufweist, wobei Cu-, Co- und ZnO-Mengen im Dotierbereich ausgenommen sind, das molare Verhältnis von ZnO zu ZrO₂ vorzugsweise 1:20-50 ist, das molare Verhältnis von Cu zu Ni 1:100-200 ist und das molare Verhältnis von Co zu Ni 1:50-200 ist.

## Revendications

1. Procédé de production d'anhydride hexahydro-3,6-méthanophtalique ayant la formule structurelle suivante : 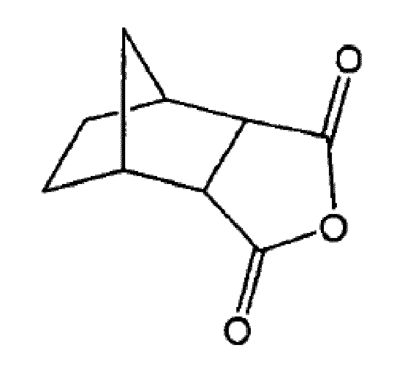 **caractérisé en ce que** le procédé comprend les étapes suivantes : ajouter de l'anhydride maléique à du C₅S mélangé progressivement en agitant, contrôler une température réactionnelle à 0 à 5°C, terminer la réaction lorsque aucun cyclopentadiène n'est détecté par détection gazeuse, séparer par centrifugeuse, vaporiser, compresser et condenser le liquide pour obtenir du C₅S décyclisé ayant de l'isoprène et du 1,3-pentadiène comme composants principaux, cristalliser le solide pour obtenir de l'anhydride nadique ; puis ajouter un catalyseur Cu-Ni/ZnO-ZrO₂ ou Co-Ni/Zno-ZrO₂ à l'anhydride nadique obtenu, effectuer une réaction d'hydrogénation catalytique pendant 4 à 6 heures dans un autoclave à haute pression à une température de 100 à 140°C et sous une pression d'hydrogène de 1 à 4 MPa, filtrer le catalyseur après refroidissement et distiller sous pression réduite pour obtenir l'anhydride hexahydro-3,6-méthanophtalique.

2. Procédé de production d'anhydride hexahydro-3,6-méthanophtalique selon la revendication 1, **caractérisé en ce que** du cyclopentadiène, du 1,3-pentadiène et de l'isoprène sont les composants principaux du C₅S mélangé.

3. Procédé de production d'anhydride hexahydro-3,6-méthanophtalique selon la revendication 1, **caractérisé en ce que**, dans le catalyseur Cu-Ni/ZnO-ZrO₂ ou Co-Ni/Zno-ZrO₂, une teneur en Ni est de 10 à 30 % en masse excluant des quantités dopantes de Cu, Co et Zno, un rapport molaire entre Zno et ZrO₂ est de préférence de 1/20-50, un rapport molaire entre Cu et Ni est de 1/100-200 et un rapport molaire entre Co et Ni est de 1/50-200.
